# FASCICULE DE BREVET EUROPEEN

(11) **EP 1 390 524 B1**
(45) Date de publication et mention de la délivrance du brevet: **11.10.2006**
(21) Numéro de dépôt: 02757748.5
(22) Date de dépôt: 29.03.2002
(51) Int. Cl.: C12Q 1/14, C12Q 1/34

(54) **MILIEUX DE DETECTION SPECIFIQUE DE STAPHYLOCOCCUS AUREUS ET PROCEDES D'IDENTIFICATION ET/OU DE DENOMBREMENT UTILISANT DE TELS MILIEUX**
SPEZIFISCHE DETEKTIONSMEDIEN FÜR STAPHYLOCOCCUS AUREUS UND IDENTIFIZIERUNGS- UND/ODER ZAEHLMETHODE UNTER VERWENDUNG BESAGTER DETEKTIONSMEDIEN
STAPHYLOCOCCUS AUREUS SPECIFIC DETECTION MEDIA AND IDENTIFYING AND/OR COUNTING METHOD USING SAME

(30) Priorité: 30.03.2001 FR 0104322
(43) Date de publication de la demande: 25.02.2004
(73) Titulaire: Biomerieux S.A., 69280 Marcy l'Etoile (FR)
(72) Inventeur: COTTE, Christine, F-38800 Saint-Savin (FR); ORENGA, Sylvain, F-01160 Neuville sur Ain (FR); RE, Andréa, F-69160 Tassin la Demi-Lune (FR); ROBICHON, Denis, 01150 BLYES (FR)
(74) Mandataire: de Zeeuw, Johan Diederick
(86) Numéro de dépôt international: PCT/FR2002/001120
(87) Numéro de publication internationale: WO 2002/079486

(56) Documents cités:
- WO-A-00/53799
- WO-A-95/20674
- WO-A-98/55644
- WO-A-99/50438
- S. BASCOMB ET AL.: "Use of enzyme tests in characterization and identification of aerobic and facultatively anaerobic Gram-positive cocci." CLINICAL MICROBIOLOGY REVIEWS., vol. 11, no. 2, avril 1998 (1998-04), pages 318-340, XP002190158 WASHINGTON, DC., US ISSN: 0893-8512 cité dans la demande
- J. A. LINDSAY ET AL.: "Identification of Staphylococcus epidermidis and Staphylococcus hominis from blood cultures by testing susceptibility to desferrioxamine." EUROPEAN JOURNAL OF CLINICAL MICROBIOLOGY & INFECTIOUS DISEASES., vol. 12, 1993, pages 127-131, XP000921267 SPRINGER, WIESBADEN, DE ISSN: 0934-9723 cité dans la demande

## Description

La présente invention concerne un milieu de détection spécifique de *Staphylococcus aureus* et/ou de discrimination des *Staphylococcus* à coagulase positive par rapport aux *Staphylococcus* à coagulase négative permettant l'isolement des bactéries du genre staphylocoque et l'identification de l'espèce *Staphylococcus aureus,* qui utilisent au moins un substrat enzymatique. Elle concerne également un procédé d'identification, et éventuellement de dénombrement, des *Staphylococcus aureus* qui utilise un tel milieu.

En 1999, le genre *Staphylococcus* regroupe quarante-trois (43) espèces et sous-espèces, dont dix-sept (17) ont été retrouvées chez l'homme. La plupart de ces espèces sont des pathogènes opportunistes chez l'homme présentant un risque élevé en cas de blessure cutanée par un traumatisme ou par implantation directe d'un produit médical. D'ailleurs, l'espèce *Staphylococcus aureus* est une bactérie qui se retrouve souvent chez des patients qui doivent recevoir des soins hospitaliers faisant intervenir des appareils tels que seringues, cathéters. Il y a donc un grand intérêt de détecter la présence de cette bactérie pathogène, de plus en plus impliquée dans les maladies nosocomiales.

Parmi les staphylocoques. *Staphylococcus aureus* est incontestablement l'espèce la plus virulente, puisqu'elle produit un nombre important de toxines et d'enzymes extracellulaires. Elle peut être à l'origine de pathologies nombreuses et variées, allant du simple panaris, jusqu'aux infections les plus sévères comme les septicémies, endocardites, pneumopathies, infections ostéoarticulaires, dont le pronostic peut être très réservé.

En outre, seules cinq (5) espèces : *Staphylococcus aureus, Staphylococcus epidermidis, Staphylococcus haemolyticus, Staphylococcus saprophyticus* et *Staphylococcus hominis* représentent au moins 1% des souches pathogènes isolées dans des centres cliniques et, à elles cinq, plus de 98% des souches de staphylocoques les plus fréquemment rencontrées. Les autres espèces sont rarement rencontrées et peu pathogènes.

En bactériologie, il est classique d'opposer l'espèce *Staphylococcus aureus,* caractérisée par la production d'une coagulase, aux autres espèces dites à coagulase négative.

Les méthodes traditionnelles pour différencier *Staphylococcus aureus* de *Staphylococcus* non *aureus* reposent sur la recherche de la coagulase libre, de la DNase et sur l'obtention d'une agglutination sur latex visant à mettre en évidence la présence du facteur d'affinité pour le fibrinogène, de la protéine A et d'antigènes capsulaires.

Il faut savoir que d'autres espèces de staphylocoques, potentiellement pathogènes, sont capables d'exprimer une coagulase.

Les espèces à coagulase positive sont les suivantes : *Staphylococcus aureus, Staphylococcus intermedius, Staphylococcus hyicus, Staphylococcus delphinis, Staphylococcus lutrae* et *Staphylococcus schleiferi.*

Il existe des techniques de culture sur milieux sélectifs pour permettre d'évaluer la présence de *Staphylococcus aureus.* Il s'agit des milieux suivants :
- Le milieu hypersalé de Chapman (à 75 % de NaCl) est généralement sélectif pour *Staphylococcus aureus* et les staphylocoques qui hydrolysent le Mannitol. Ces bactéries font virer le milieu de rouge au jaune. Certains micro-organismes et les entérocoques du groupe D, en particulier, peuvent produire la même réaction sur le milieu ; il est donc nécessaire alors de vérifier la catalase (négative pour les streptocoques).
- Le milieu de Baird Parker (contenant du Tellurite de potassium et du Chlorure de lithium comme agents sélectifs) est utilisé pour isoler et dénombrer les staphylocoques à coagulase positive dans les produits alimentaires et permet de mettre en évidence l'activité de la coagulase. Sur ce milieu les colonies de *Staphylococcus aureus,* et d'autres espèces à coagulase positive apparaissent avec un centre noir entouré d'un halo opaque. D'autres micro-organismes peuvent se développer sur ce milieu. Il s'agit principalement des groupes suivants :
   ■ Cocci Gram + : des genres *Enterococcus* et *Listeria,*
   ■ Bacille Gram - : des genres *Proteus* et *Pseudomonas.*

En fait, la révélation des *Staphylococcus aureus,* qui utilise la technique du milieu hyper salé de Chapman, manque de sensibilité (pouvoir de mettre en évidence l'espèce recherchée lorsque celle-ci est présente en faible quantité dans un échantillon biologique à tester) et surtout de spécificité (pouvoir de détecter l'espèce recherchée dans l'échantillon biologique à tester contenant d'autres espèces).

De même, la révélation des *Staphylococcus aureus,* qui utilise la technique du milieu Baird-Parker, manque de sensibilité et de spécificité. Ainsi, certaines souches n'appartenant pas à l'espèce *Staphylococcus aureus,* en particulier *Staphylococcus schleiferi* et *Staphylococcus saprophyticus,* peuvent également donner des colonies entourées d'une auréole d'éclaircissement. Il peut arriver aussi que certaines souches de *Staphylococcus aureus* n'expriment pas l'activité enzymatique recherchée ou ne se développent pas sur le milieu, car ils peuvent être présents en trop faible quantité dans les échantillons biologiques et/ou inhibées par les composants trop sélectifs du milieu.

On comprend donc que cette révélation avec les milieux Baird-Parker et Chapman, ne donnent qu'un diagnostic présomptif et nécessitent d'autres tests de confirmation. Or, les manipulations supplémentaires, nécessaires à l'identification de *Staphylococcus aureus,* augmentent la durée et le coût des analyses. Elles nécessitent une multitude de réactifs et l'intervention d'un personnel qualifié.

Il est également possible d'utiliser des substrats à base de glucoside, ce qui permet de détecter, selon la configuration des molécules utilisées, soit une activité α-glucosidase, soit une activité β-glucosidase.

L'utilisation d'α-glucosides pour l'identification des différentes espèces de staphylocoques est déjà connue. Cependant, l'étude de l'acidification de ces sucres par les différentes espèces de staphylocoques ne permet pas actuellement une identification simple de *Staphylococcus aureus,* car elle est soit trop peu spécifique (dans le cas du Maltose, du Turanose, du Saccharose, du Tréhalose par exemple), soit trop peu sensible (dans le cas du Méthyl-α-glucoside, du Raffinose, du Turanose par exemple) (Bascomb S. and Manafi M. 1998. Use of enzyme tests in characterization and identification of aerobic and facultatively anaerobic Gram positive cocci. Clin. Microbiolol. Rev. 11 : 318-340.) (Kloos WE. et al. 1982. Identification of *Staphylococcus* Species with the API Staph -IDENT. System. J. Clin. Microbiol. 16 (3) : 509-516.). Cet état de fait n'a pas tendance à pousser les chercheurs à travailler sur ce type d'activité α-glucosidase.

Par contre, des publications récentes font état de l'intérêt de travailler avec l'activité β-glucosidase. C'est par exemple le cas du brevet EP-B-0.741.797, qui décrit un procédé de différentiation sur un milieu de culture sélectif de bactéries de genre *Staphylococcus* des autres bactéries par l'utilisation de deux substrats chromogéniques à base d'Indoxyle permettant de détecter l'activité phosphatase des staphylocoques et l'activité β-glucosidase des autres genres de bactéries.

Par rapport à la détection de staphylocoques sur un milieu gélifié, selon ce brevet, permettant seulement la différenciation de staphylocoques de bactéries d'autres genres, notre invention permet donc de distinguer les *Staphylococcus aureus* des autres bactéries du genre *Staphylococcus*.

Dans un article : « Evaluation of CHROMagar Staph. aureus, a new chromogenic medium, for isolation and presumptive identification of *Staphylococcus aureus* from human clinical specimens. » de Gaillot O. *et al.* 2000. J. Clin. Microbiol. 38, 4, 1587-1591, on décrit et évalue un milieu de culture chromogénique CHROMagar (marque déposée) Staph. aureus permettant l'isolement des staphylocoques et l'identification de *Staphylococcus aureus* à l'aide de substrats chromogéniques qui procurent une coloration mauve de cette dernière espèce. Les autres espèces du même genre sont alors détectées par la coloration bleue ou incolore, en théorie. On utilise essentiellement les activités β-glucosidase, β-glucuronidase, β-galactosidase et phosphatase, ainsi qu'un inhibiteur, la Déféroxamine, qui permet la différenciation de *Staphylococcus aureus* et *Staphylococcus epidermidis.* Une demande de brevet a d'ailleurs été déposée sur ce sujet, WO-A-00/53799. Cette demande de brevet propose un milieu de détection de *Staphylococcus aureus* qui couple au moins les deux agents chromogènes suivants :
- 5-Bromo-6-chloro-3-indoxyl phosphate, activité phosphatase, et
- 5-Bromo-4-chloro-3-indoxyl glucoside, activité β-glucosidase,
déjà décrits dans la demande de brevet EP-B-0.741.797, décrite plus haut. En fait, ce nouveau document ajoute aux chromogènes simplement et essentiellement un inhibiteur de croissance, appelé Déféroxamine. Ce dernier permet de mieux discriminer les *Staphylococcus aureus* des *Staphylococcus epidermidis.* Or la Déféroxamine est déjà bien connue comme inhibiteur de croissance des *Staphylococcus epidermidis* mais pas pour les autres espèces de staphylocoque (Lindsay J.A., Aravena-roman M.A., Riley T.V. - Identification of *Staphylococcus epidermidis* and *Staphylococcus hominis* from blood cultures by testing susceptibility to desferrioxamine - European J. of Clin. Microbiol. et Inf. Diseases - 1993, vol. 12, pages 127-131).

Par rapport au milieu CHROMagar (marque déposée) Staph. aureus, notre milieu permet une différentiation plus aisée des *Staphylococcus aureus* des *Staphylococcus epidermidis,* les deux espèces produisant des colonies de la même couleur sur le milieu CHROMagar (marque déposée). Ceci est dû au manque de spécificité des substrats de phosphatase qui sont positifs pour les *Staphylococcus aureus* et les *Staphylococcus epidermidis* et au fait que l'inhibition de ces derniers par la Déféroxamine n'est que partielle.

Il faut noter que selon notre invention, en détectant uniquement l'activité α-glucosidase, on peut déjà séparer *Staphylococcus aureus* de *Staphylococcus epidermidis* et *Staphylococcus saprophyticus* qui sont trois des principales espèces de staphylocoques isolées en clinique.

En fait et contre toute attente, les inventeurs ont démontré que des substrats à base d'α-glucoside pouvaient permettre une identification sensible et spécifique de *Staphylococcus aureus.* En fait, par le choix de conditions opérationnelles et/ou de substrats particuliers d'α-glucosidase et/ou de coupler au moins un deuxième substrat enzymatique permettant de définir un milieu réactionnel, il est possible de :
- discriminer entre les principales espèces de staphylocoques à coagulase positive les plus fréquemment isolées (*Staphylococcus aureus* et *Staphylococcus intermedius* essentiellement) et les staphylocoques à coagulase négative les plus présents (*Staphylococcus epidermidis, Staphylococcus haemolyticus, Staphylococcus saprophyticus* essentiellement), et/ou
- identifier spécifiquement des *Staphylococcus aureus.*

Par exemple, un gain de spécificité et dans l'aspect pratique de l'invention est apporté par l'adjonction d'au moins un deuxième substrat enzymatique. Ainsi, ce gain est obtenu par la détection, d'une part, d'une activité α-glucosidase, activité fortement exprimée par les *Staphylococcus aureus* et très faiblement par les autres espèces de staphylocoque, et d'autre part, d'une activité β-glucuronidase et/ou ß-galactosidase et/ou β-glucosidase exprimée(s) par la plupart des staphylocoques mais pas par les *Staphylococcus aureus* en fonction des substrats utilisés. Cette deuxième activité permet de mieux différencier les *Staphylococcus aureus* des autres espèces du genre *Staphylococcus.*

A cet effet, la présente invention concerne un milieu de détection de *Staphylococcus aureus* et/ou de Staphylocoques à coagulase positive, qui comporte un milieu de culture de *Staphylococcus aureus* et au moins un substrat enzymatique permettant de mettre en évidence une activité α-glucosidase tel que défini dans les revendications.

Le substrat enzymatique permettant de mettre en évidence une activité α-glucosidase est un agent chromogène ou fluorescent.

Selon une première variante de réalisation, l'agent chromogène ou fluorescent permettant de mettre en évidence une activité α-glucosidase est à base d'Indoxyle ou d'Umbelliférone, pour permettre la détection de *Staphylococcus aureus* et des Staphylocoques à coagulase positive.

Ce milieu, selon la première variante de réalisation, utilise comme agent(s) chromogène(s) :
- 6-Bromo-3-indoxyl-α-D-glucoside,
- 5-Bromo-4-chloro-3-indoxyl-α-D-glucoside,
- 5-Bromo-4-chloro-3-indoxyl-N-méthyl-α-D-glucoside,
- 6-Chloro-3-indoxyl-α-D-glucoside, et/ou
- 4-Méthylumbelliféryl-α-D-glucoside.

Selon une seconde variante de réalisation, l'agent chromogène permettant de mettre en évidence une activité α-glucosidase est à base de Naphtole, pour permettre la détection de *Staphylococcus aureus.*

Ce milieu, selon la seconde variante de réalisation, utilise comme agent chromogène : 2-Naphtyl-α-D-glucopyranoside.

Selon un mode de réalisation, le milieu utilise au moins deux substrats enzymatiques différents, préférentiellement deux agents chromogènes, l'un permettant de mettre en évidence une activité α-glucosidase et l'autre permettant de détecter une activité osidase (β-glucosidase et/ou β-glucuronidase et/ou β-galactosidase) et/ou estérase (estérase/lipase et/ou phosphatase et/ou sulfatase) et/ou peptidase et/ou coagulase.

De plus, le milieu utilise deux agents chromogènes, choisis parmi les couples suivants :
- 5-Bromo-4-chloro-3-indoxyl-N-méthyl-α-D-glucoside associé au 5-Bromo-6-chloro-3-indoxyl-β-D-galactoside, ou
- 5-Bromo-4-chloro-3-indoxyl-N-méthyl-α-D-glucoside associé au 6-Chloro-3-indoxyl-β-D-glucuronide, ou
- 6-Chloro-3-indoxyl-α-D-glucoside associé au 5-Bromo-4-chloro-3-indoxyl-β-D-glucuronide, ou
- 6-Chloro-3-indoxyl-α-D-glucoside associé au 5-Bromo-4-chloro-3-indoxyl-β-D-glucoside.

Le milieu utilise au moins un inhibiteur qui privilégie la croissance des bactéries du genre *Staphylococcus,* tel que Chlorure de lithium (LiCl), Azide de sodium (NaN₃), Colistine, Amphotéricine, Aztréonam, Colimicine, Chlorure de sodium (NaCl) et Déféroxamine.

Selon une variante de réalisation, le milieu utilise un mélange d'inhibiteurs, comprenant quatre inhibiteurs, qui privilégie la croissance des bactéries du genre *Staphylococcus,* qui sont :
- LiCl,
- O/129,
- Aztréonam, et
- Amphotéricine.

Selon une autre variante, le milieu utilise du maltose, préférentiellement à une concentration comprise entre 100 et 300 mg/l.

La présente invention concerne également une méthode d'identification de bactérie(s) de l'espèce *Staphylococcus aureus* dans un échantillon, contenant au moins une espèce de bactérie, qui comprend les étapes suivantes :
- inoculer un milieu, tel que décrit ci-dessus, avec tout ou partie de l'échantillon, et
- incuber le milieu inoculé pour produire des colonies bactériennes de taille suffisante pour observer la coloration de chaque agent chromogène ou la fluorescence de chaque agent fluorescent, ayant réagi, utilisé dans ledit milieu.

Cette méthode peut permettre en outre le dénombrement de bactérie(s) de l'espèce *Staphylococcus aureus* dans l'échantillon, dans ce cas, on effectue une troisième étape qui consiste à énumérer les colonies identifiées par une coloration ou une fluorescence en relation avec les bactéries de l'espèce *Staphylococcus aureus.*

### Expérience 1 : Evaluation de différents substrats d'α-glucosidase constitués par différents marqueurs chromogéniques à base de dérivés d'Indoxyl :

Les milieux ci-après ont été préparés dans une base contenant :
- 11 grammes par litre (g/l) de peptone,
- 0,65 g/l de tampon TRIS, et
- 14 g/l d'agar,
en combinaison avec les substrats chromogéniques d'α-glucosidase décrits dans le tableau 1 suivant :

**Tableau 1 : Abréviations des substrats chromogéniques d'α-glucosidase utilisés**

| **Substrat chromogénique** | **Abréviation utilisée** |
|---|---|
| 5-Bromo-4-chloro-3-indoxyl-α-D-glucoside | **5-Bromo-4-chloro-3-indoxyl-α-D-glucoside** |
| 6-Chloro-3-indoxyl-α-D-glucoside | **Rose-a-GLU** |
| 6-Bromo-3-indoxyl-α-D-glucoside | **Red «A» a-GLU** |
| 5-Bromo-4-chloro-3 -indoxyl-N-méthyl-α-D-glucoside | **Green «A» a-GLU** |

Les substrats de l'α-glucosidase sont solubilisés dans le Diméthylsulfoxide à une concentration de 200 g/l. Un volume suffisant est ajouté aux quatre milieux en surfusion pour obtenir une concentration finale en substrat à 100 mg/l. Des micro-organismes issus de la collection interne de bioMérieux ont été ensemencés sur chacun des milieux par isolement en trois cadrans à partir d'une suspension à 0,5 MacFarland. Néanmoins, des résultats obtenus avec des micro-organismes issus de collections disponibles au public, ATCC ou NCTC par exemple, donneraient des résultats identiques. Les boîtes ont été incubées à 37°C pendant 48 heures. Les colonies formées ont été examinées visuellement après un temps d'incubation de 24 et 48 heures. La coloration ainsi que l'intensité de cette coloration ont été notées.

Dans le tableau 2 ci-dessous, comme dans les tableaux qui suivront, C représente la coloration des colonies après incubation, I représente l'Intensité de cette coloration, le symbole « - » est synonyme d'une absence de couleur ou d'intensité, enfin TI définit les temps d'incubation. A noter que l'intensité de la coloration est une échelle arbitraire mais qui est commune à l'ensemble des échantillons biologiques et des milieux testés. Cette échelle est valable pour cette expérience ainsi que pour toutes les expériences qui suivront. Elle peut être définie de la manière suivante :
- 0 correspond à une absence d'activité,
- 0,1 correspond à la présence d'une trace de coloration,
- 0,5 correspond à la présence d'une coloration très pâle,
- 1 correspond à la présence d'une coloration nette de faible intensité,
- 1,5 Correspond à la présence d'une coloration intermédiaire aux colorations 1 et 2,
- 2 correspond à la présence d'une coloration franche d'intensité moyenne,
- 2,5 correspond à la présence d'une coloration intermédiaire aux colorations 2 et 3,
- 3 correspond à la présence d'une coloration intense,
- 3,5 correspond à la présence d'une coloration intermédiaire aux colorations 3 et 4,
- 4 correspond à la présence d'une coloration très intense.

Les résultats sont présentés dans le tableau 2 ci-dessous :

**Tableau 2 : Evaluation de différents substrats d'α-glucosidase constitués par différents marqueurs chromogéniques à base de dérivés d'Indoxyl**

| | **Milieu** | **X-a-GLU** | | **Rose-a-GLU** | | **Red "A" a-GLU** | | **Green "A" a-GLU** | |
|---|---|---|---|---|---|---|---|---|---|
| Souches | TI | C | I | C | I | C | I | C | I |
| *S. aureus* | 24 h | turquoise | 1,5 | rose | 2 | rose | 2 | vert | 1,5 |
| *070* | 48h | turquoise | 1,5 | rose | 2 | rose | 2,5 | vert | 2,5 |
| *S. aureus* | 24 h | turquoise | 2 | rose | 1,5 | rose | 2 | vert | 2 |
| *276* | 48 h | turquoise | 2 | rose | 1,5 | rose | 2 | vert | 2 |
| *S. aureus* | 24 h | turquoise | 1 | rose | 1 | rose | 0,5 | vert | 1,5 |
| *004* | 48 h | turquoise | 2 | rose | 1 | rose | 2,5 | vert | 2 |
| *S. intermedius* | 24 h | turquoise | 0,5 | - | 0 | - | 0 | vert | 1,5 |
| 075 | 48 h | turquoise | 2 | rose | 0,5 | rose | 1,5 | vert | 1,5 |
| *S. saprophyticus* | 24 h | - | 0 | - | 0 | - | 0 | - | 0 |
| *064* | 48 h | turquoise | 0,5 | - | 0 | - | 0 | - | 0 |
| *S. epidermidis* | 24 h | - | 0 | - | 0 | - | 0 | - | 0 |
| *009* | 48h | turquoise | 1 | - | 0 | - | 0 | - | 0 |
| *S. xylosus* | 24 h | turquoise | 0,5 | - | 0 | - | 0 | - | 0 |
| *008* | 48h | turquoise | 1,5 | rose | 0,5 | rose | 2,5 | vert | 1 |

Pour le substrat 5-Bromo-4-chloro-3-indoxyl-α-D-glucoside, on obtient un écart d'intensité de coloration entre les souches de staphylocoques à 24 heures, qui permet d'identifier les *Staphylococcus aureus* et de les distinguer des autres espèces du genre *Staphylococcus,* A 48 heures, un doute peu exister entre *Staphylococcus aureus* et *Staphylococcus xylosus.*

Pour les trois autres substrats, la durée d'incubation n'a pas d'influence, en ce sens que l'écart d'intensité de coloration, à 24 heures comme à 48 heures, permet l'identification des *Staphylococcus aureus* et leur distinction par rapport aux autres espèces du genre *Staphylococcus.*

On remarque toutefois que les écarts d'intensité entre les souches de *Staphylococcus aureus* et les souches de *Staphylococcus xylosus* (à coagulase négative) sont plus significatifs avec les trois substrats 6-Chloro-3-indoxyl-α-D-glucoside, 6-Bromo-3-indoxyl-α-D-glucoside et 5-Broino-4-chloro-3-indoxyl-N-méthyl-α-D-glucoside qui donnent un meilleur contraste entre la couleur du marqueur et celle du milieu et sont plus spécifiques que le 5-Bromo-4-chloro-3-indoxyl-α-D-glucoside.

### Expérience 2 : Différentiation des Staphylococcus aureus par rapport aux espèces du même genre par la détection directe de l'activité α-glucosidase et d'une autre activité osidase dans un milieu gélifié :

Les milieux ci-après ont été préparés dans une base contenant :
- 11 gramme par litre (g/l) de peptone,
- 0,65 g/l de tampon TRIS, et
- 14 g/l d'agar,
associée à une combinaison de deux substrats chromogéniques dont l'un permet de détecter une activité α-glucosidase. Ces substrats complexes sont présentés dans le tableau 3 qui suit :

**Tableau 3 : Abréviation de différentes combinaisons de substrats chromogéniques d'α-glucosidase et de substrats chromogéniques spécifiques d'autres activités enzymatiques**

| **Abréviation** | **Substrat α-glucosidase** | **Substrat associé (activité détectée)** |
|---|---|---|
| A | 5-Bromo-4-chloro-3-indoxyl-N-methyl-α-D-glucoside | 6-Chloro-3-indoxyl-(β-D-glucuronide (β-glucuronidase) |
| B | 5-Bromo-4-chloro-3-indoxyl-N-methyl-α-D-glucoside | 6-Chloro-3-indoxyl-β-D-galactoside (β-galactosidase) |
| C | 6-Chloro-3-indoxyl-α-D-glucoside | 5-Bromo-4-chloro-3-indoxyl-β-D-glucoside (β-glucosidase) |
| D | 6-Chloro-3-indoxyl-α-D-glucoside | 5-Bromo-4-chloro-3-indoxyl-β-D-glucuronide (β-glucuronidase) |

Une solution mère de chaque substrat d'α-glucosidase à 200 g/l est préparée dans le Diméthylsulfoxide. Un volume suffisant est ajouté aux quatre milieux en surfusion pour obtenir une concentration finale pour chaque substrat d'α-glucosidase de 100 mg/l. En même temps, un volume suffisant de substrat de chacune des autres activités osidases, décrites ci-dessus, est ajouté aux quatre milieux à une concentration comprise entre 50 et 200 mg/l selon le substrat. Des micro-organismes issus de la collection interne de bioMérieux ont été ensemencés sur chacun des milieux par isolement en trois cadrans à partir d'une suspension à 0,5 MacFarland. Les boîtes ont été incubées à 37°C pendant 48 heures. Les colonies formées ont été examinées visuellement après 24 et 48 heures d'incubation. La coloration ainsi que l'intensité de cette coloration ont été notées.

Les résultats sont présentés dans le tableau 4 ci-dessous :

**Tableau 4 : Evaluation de différents combinaisons de substrats**

| | **Milieu** | **A** | | **B** | | **C** | | **D** | |
|---|---|---|---|---|---|---|---|---|---|
| Souches | TI | C | I | C | I | C | I | C | I |
| *S. aureus* | 24 h | vert | 1,5 | vert | 1,5 | rose | 1,5 | rose | 2 |
| *070* | 48 h | vert | 1,5 | vert | 1,5 | rose | 3 | rose | 3 |
| *S. aureus* | 24 h | vert | 2 | vert | 2 | rose | 1,5 | rose | 1,5 |
| 2*76* | 48 h | vert | 3 | vert | 3 | rose | 3,5 | rose | 2 |
| *S. aureus* | 24 h | vert | 1,5 | vert | 1,5 | rose | 1,5 | rose | 1,5 |
| *004* | 48 h | vert | 1,5 | vert | 1,5 | rose | 2 | rose | 2 |
| *S. intermedius* | 24 h | vert | 0,5 | gris | 0,5 | rose | 0,5 | rose | 0 |
| *075* | 48 h | vert | 1,5 | mauve | 2 | rose | 1,5 | rose | 1,5 |
| *S. haemolyticus* | 24 h | - | 0 | - | 0 | rose | 0,5 | rose | 0,5 |
| *131* | 48 h | - | 0 | - | 0 | rose | 0,5 | rose | 0,5 |
| *S. saprophyticus* | 24 h | - | 0 | rose | 2 | - | 0 | - | 0 |
| *064* | 48 h | - | 0 | rose | 2,5 | rose | 0,5 | rose | 0,5 |
| *S. epidermidis* | 24 h | - | 0 | - | 0 | - | 0 | - | 0 |
| *009* | 48 h | - | 0 | - | 0 | rose | 0,5 | rose | 0,5 |
| *S. xylosus* | 24 h | rose | 1,5 | mauve | 0,5 | turquoise | 3 | turquoise | 1,5 |
| *008* | 48 h | mauve | 3,5 | mauve | 1,5 | turquoise | 3 | turquoise | 2,5 |

Les résultats ci-dessus montrent qu'il est possible de distinguer les *Staphylococcus aureus* des autres espèces, les plus fréquemment rencontrées (*Staphylococcus epidermidis, Staphylococcus saprophyticus,* etc.) après 24 heures d'incubation avec tous les couples de substrats chromogéniques testés.

Chaque couple A, B, C et D de substrats étudié par la gamme de couleurs et/ou par l'intensité de couleur permet de distinguer les différentes espèces de staphylocoques. D'une part, la différence de coloration permet de repérer *Staphylococcus xylosus* sans ambiguïté, et d'autre part les niveaux d'intensité de la couleur les plus forts, obtenus pour les *Staphylococcus aureus,* permettent de les différencier des autres espèces du même genre ayant la même coloration.

Seule l'espèce *Staphylococcus intermedius* peut poser problème à 48 heures avec le couple de substrat A, néanmoins il n'y a aucune ambiguïté à 24 heures d'incubation, ce qui est plus intéressant car plus la durée d'identification est courte plus le milieu est performant. Il s'agit d'une espèces de staphylocoque à coagulase positive qui n'est pas distinguée de *Staphylococcus aureus* par la plupart des méthodes d'identification notamment sur les milieux Chapman, Baird Parker et CHROMagar (marque déposée) Staph aureus.

### Expérience 3 : Etude comparative d'un de nos milieux contenant un mélange de substrats décrit à l'Expérience 2 vis-à-vis de milieux disponibles dans le commerce :

Les milieux de culture suivants, révélateurs de la présence de *Staphylococcus aureus* dans un prélèvement biologique peuvent être obtenus chez les sociétés suivantes :
- milieu CHROMagar Staph aureus (référence TA600) auprès de la société CHROMagar située à Paris en France, et
- milieux Chapman (référence 43311) et Baird Parker (référence 43521) auprès de la société bioMérieux située à Marcy l'Etoile en France.

Les abréviations des milieux présentés dans le tableau 5 ci-dessous sont les suivantes :
- A correspond au milieu contenant le mélange A décrit à l'Expérience 2,
- B correspond au milieu CHROMagar Staph aureus utilisé pour isoler les staphylocoques et identifier l'espèce *Staphylococcus aureus* par une coloration rose des colonies, les autres espèces de staphylocoques donnent une couleur turquoise ou incolore,
- C correspond au milieu Chapman utilisé pour isoler *Staphylococcus aureus* et les staphylocoques qui hydrolysent le mannitol, ces bactéries font virer l'indicateur coloré, présent dans le milieu, du rouge au jaune, et
- D correspond au milieu Baird Parker utilisé pour isoler et caractériser *Staphylococcus aureus* qui produit en 24 heures des colonies caractéristiques à centre noir entouré d'une zone d'éclaircissement, dit halo, correspondant à la détection d'une coagulase.

Dans les milieux décrits ci-dessus ont été testées trente-six (36) souches appartenant à douze (12) espèces de staphylocoques, les plus fréquemment rencontrées en analyse clinique. Ces micro-organismes issus de la collection interne de bioMérieux ont été ensemencés sur chacun des milieux par isolement en trois cadrants à partir d'une suspension à 0,5 McFarland. Les boîtes ont été incubées à 37°C pendant 48 heures. Les colonies formées ont été examinées visuellement après 24 et 48 heures d'incubation. La coloration des colonies sur notre milieu et le milieu chromogénique CHROMagar Staph aureus ont été notées selon le procédé et l'échelle décrit dans l'exemple 1. Pour la lecture du milieu Chapman, le symbole « - » correspond à une absence de virage de l'indicateur coloré, la gélose reste rouge autour des colonies (souches mannitol -) et le symbole « + » correspond à un virage de l'indicateur coloré, la gélose devient jaune autour des colonies (souche mannitol +). Pour la lecture du milieu Baird Parker, le symbole «-» correspond à une absence de halo d'éclaircissement autour des colonies (souche coagulase -) et le symbole « + » correspond à la présence d'un halo d'éclaircissement autour desdites colonies (souche coagulase +).

Dans le tableau 5 ci-dessous, la coloration correspond à la coloration des colonies après incubation, les initiales « C.M. » représente la coloration du milieu Chapman autour des colonies après incubation, le terme « Halo » représente la zone d'éclaircissement du milieu Baird Parker autour des colonies après incubation, enfin le symbole « n° » représente le nombre de souches par espèces identifiées, selon le milieu, par la coloration spécifique ou le halo.

**Tableau 5 : Comparaison d'un milieu selon l'invention avec les milieux disponibles dans le commerce.**

| | **Milieu** | **A** | | **B** | | **C** | | **D** | |
|---|---|---|---|---|---|---|---|---|---|
| **Souches** | **TI** | **Coloration** | **n°** | **Coloration** | **n°** | **C. M.** | **n°** | **Halo** | **n°** |
| *S. aureus* | 24 h | vert | 9 | rose | 9 | + | 9 | + | 3 |
| *(9)* | 48 h | vert | 9 | rose | 9 | + | 9 | + | 5 |
| *S. intermedius* | 24 h | vert | 2 | rose | 2 | - | - | + | 2 |
| *(2)* | 48 h | vert | 2 | rose | 2 | - | - | + | 2 |
| *S. haemolyticus* | 24 h | - | - | - | - | + | 2 | - | - |
| *(3)* | 48h | - | - | - | - | + | 2 | - | - |
| *S. saprophyticus* | 24 h | - | - | turquoise | 3 | + | 1 | - | - |
| *(3)* | 48h h | - | - | turquoise | 3 | + | 1 | - | - |
| *S. epidermidis* | 24 h | - | - | rose | 4 | - | - | - | - |
| *(5)* | 48 h | - | - | rose | 4 | - | - | - | - |
| *S. hominis* | 24 h | - | - | turquoise | 1 | - | - | - | - |
| *(2)* | 48h | - | - | turquoise | 1 | - | - | - | - |
| *S. warneri* | 24 h | - | - | - | - | + | 1 | - | - |
| *(2)* | 48 h | - | - | - | - | + | 1 | - | - |
| *S. schleiferi* | 24 h | - | - | rose | 2 | - | - | + | 2 |
| *(2)* | 48 h | - | - | rose | 2 | - | - | + | 2 |
| *S. cohnii* | 24 h | rose | 1 | turquoise | 1 | + | 2 | - | - |
| *(2)* | 48 h | rose | 1 | turquoise | 2 | + | 2 | - | - |
| *S. xylosus* | 24 h | rose mauve | 2 | turquoise | 2 | + | 1 | - | - |
| *(2)* | 48 h | | 2 | turquoise | 2 | + | 2 | - | - |
| *S. capitis* | 24 h | - | - | - | - | + | 1 | - | - |
| (*2*) | 48 h | - | - | - | - | + | 2 | - | - |
| *S. hyicus* | 24 h | - | - | rose | 2 | - | - | - | - |
| (*2*) | 48 h | - | - | mauve | 2 | - | - | + | 2 |

Sur le milieu, selon l'invention, et sur les milieux CHROMagar Staph aureus et Chapman toutes les souches de *Staphylococcus aureus* ont été identifiées. De plus, les souches de *Staphylococcus intermedius,* espèce à coagulase positive, sont identifiées sur notre milieu, selon l'invention, comme sur le milieux CHROMagar Staph aureus et Baird-Parker. Les résultats montrent aussi qu'aucun faux positif n'a été identifié sur le milieu selon l'invention et sur le milieu Baird Parker. On note également que sur le milieu CHROMagar Staph aureus et sur le milieu hypersalé Chapman (à 75 % de NaCl), il n'est pas possible de distinguer les *Staphylococcus aureus* de certaines espèces à coagulase négative. Il est donc nécessaire de réaliser des tests complémentaires visant à mettre en évidence la présence de ces *Staphylococcus aureus.*

Cette expérience met en évidence la spécificité et la fiabilité du procédé selon l'invention.

### Expérience 4 : Evaluation de différentes conditions opérationnelles avec un milieu contenant un substrat d'α-glucosidase à base de dérivés naphtol :

L'expérience ci-après a été réalisée dans un milieu liquide et en présence de substrats chromogéniques à base de dérivés Naphtole.

Le milieu et les substrats sont repartis dans des galeries API (marque déposée) produites par la société bioMérieux située à Marcy l'Etoile en France.

Ces galeries constituent une méthode semi-quantitative de recherche d'activités enzymatiques de micro-organismes et permettent d'étudier rapidement et simultanément dix-neuf activités enzymatiques. Chaque galerie comporte vingt cupules dans lesquelles sont réparties les solutions contenant les substrats enzymatiques. Les tests enzymatiques se réalisent en répartissant de 65 µl de suspension bactérienne à 5-6 McFarland dans chaque cupule.

L'intensité de coloration pour chaque cupule est examinée visuellement après 4 heures d'incubation à 37°C et après l'addition de réactifs appropriés : le ZYM A (réf. : 70470) et le ZYM B (réf. : 70480) qui peuvent être obtenus auprès de bioMérieux déjà évoqué précédemment. L'intensité de coloration sera directement proportionnelle à la quantité de substrat hydrolysé par l'enzyme.

Les valeurs de l'intensité de coloration sont ensuite notée sur une fiche de résultats selon le classement suivant: la note 0 correspond à une réaction négative, la note 5 à une réaction d'intensité maximale, les notes 1, 2, 3 et 4 indiquent des niveaux de coloration et donc d'activité enzymatique intermédiaires. De plus amples informations sur cette notation peuvent être trouvées dans l'échelle de lecture API fournit avec les galeries du même nom.

Comme dans les expériences 1 et 2, les micro-organismes sont issus de la collection interne de bioMérieux.

Les activités enzymatiques recherchées et les substrats de dérivés naphtol correspondant sont décrits dans le tableau 6 suivant :

**Tableau 6 : Liste des activités enzymatiques recherchées à l'aide des substrats chromogènes constitués de dérivés Naphtole.**

| N° | ENZYME RECHERCHE | SUBSTRAT |
|---|---|---|
| 1 | Témoin | Néant |
| 2 | Phosphatase alcaline | 2-Naphtyl-phosphate |
| 3 | α-galactosidase | 6-Bromo-2-naphtyl-α-D-galactopyranoside |
| 4 | β-galactosidase | 2-Naphtyl-β-D-galactopyranoside |
| 5 | β-glucuronidase | Naphtol-β-D-glucuronide |
| 6 | α-glucosidase | 2-Naphtyl-α-D-glucopyranoside |
| 7 | β-glucosidase | 6-Bromo-2-naphtyl-β-D- glucopyranoside |

Les résultats des activités enzymatiques, sont rassemblés dans le tableau 7 suivant :

**Tableau 7 : Identification des souches de Staphylococcus aureus positive par la détection de l'activité α-glucosidase par rapport à d'autres espèces de staphylocoques.**

| N° | *S. aureus* | *S. saprophyticus* | *S. epidermidis* | *S. haemolyticus* | *S. intermedius* |
|---|---|---|---|---|---|
| | 276 | 009 | 103 | 137 | 075 |
| 1 | Incolore | Incolore | Incolore | Incolore | Incolore |
| 2 | 2 | 1 | 3 | 0 | 4 |
| 3 | 0 | 0 | 0 | 0 | 0 |
| 4 | 0 | 3 | 0 | 0 | 3 |
| 5 | 0 | 0 | 0 | 0 | 0 |
| 6 | 5 | 1 | 1 | 2 | 1 |
| 7 | 0 | 0 | 0 | 0 | 0 |

D'après les résultats du tableau 7 ci-dessus, seule la détection de l'activité α-glucosidase à l'aide du substrat 2-Naphtyl-α-D-glucopyranoside, fortement exprimée par la souche de *Staphylococcus aureus,* permet de distinguer cette espèce, à coagulase positive, des autres espèces de staphylocoques. Pour les autres activités enzymatiques les écarts d'intensité de coloration entre la souche de *Staphylococcus aureus* et les souches des autres espèces sont beaucoup moins significatifs et rendent difficile la distinction entre les différentes espèces. A noter, que dans le cas d'un substrat chromogène à base de Naphtole, la distinction d'un Staphylocoque à coagulase positive comme *Staphylococcus intermedius* n'est pas possible. Les autres Staphylocoques *Staphylococcus saprophyticus, Staphylococcus epidermidis* et *Staphylococcus haemolyticus* sont à coagulase négative.

### Expérience 5 : Détection de l'α-glucosidase avec d'autres substrats non-chromogènes, tels qu'un substrat fluorescent :

Les milieux ci-après ont été préparés dans la base du milieu décrit dans l'expérience 1 mais en absence d'agar et en présence d'un substrat fluorescent en lieu et place d'un substrat chromogène. Afin de souligner l'intérêt de l'utilisation d'un substrat fluorescent pour la mise en évidence des espèces de staphylocoques à coagulase positive, quatre substrats fluorescents, décrits dans le tableau 8, spécifiques des activités enzymatiques suivantes ont été comparés :

**Tableau 8 : Abréviations des substrats fluorescents testés.**

| SUBSTRAT FLUORESCENT | ABREVIATION UTILISEE | ACTIVITE ENZYMATIQUE |
|---|---|---|
| 4-Methylumbelliferyl-α-D-glucoside | 4-MU-α-GLU | **α-glucosidase** |
| 4-Methylumbelliferyl-β-D-glucoside | 4-MU-β-GLU | β-glucosidase |
| 4-Methylumbelliferyl-β-D-galactoside | 4-MU-β-GAL | β-galactosidase |
| 4-Methylumbelliferyl-β-D-glucuronide | 4-MU-β-GUR | β-glucuronidase |

Les substrats sont solubilisés dans le solvant utilisé lors des expériences 1 et 2, la concentration finale de chaque substrat est de 200 mg/l de milieu.

Les différents milieux liquides sont répartis dans les cupules d'un support de la taille d'une carte bancaires spécifique du système automatisé VITEK 2 (marque déposée) de la société bioMérieux. Le système VITEK 2 avec étuve intégrée gère toutes les étapes, de l'inoculum au rendu des résultats. Seule la préparation de l'inoculum à 0,5 McFarland est réalisée par l'opérateur, à l'aide d'un néphélomètre, pour chacune des souches testées directement dans des tubes spécifiques du système. Le système automatisé permet la lecture, de la croissance bactérienne par néphélométrie, et de l'activité enzymatique par la fluorescence à des intervalles réguliers de 15 minutes et pendant une période de 19 heures.

Comme dans les expériences 1, 2 et 4, les souches étudiées font partie de la collection interne de bioMérieux.

Les résultats, rassemblés dans le tableau 9 ci-après, représentent le temps en heures d'incubation nécessaire pour avoir le niveau maximal de fluorescence mesurable par l'appareil :

**Tableau 9 : Tableau récapitulatif des résultats en heures d'incubation nécessaires pour l'obtention du niveau maximal de fluorescence de chaque activité enzymatique pour chacune des souches.**

| ESPECES | **4-MU-α-GLU** | 4-MU-β-GLU | 4-MU-β-GUR | 4-MU-β-GAL |
|---|---|---|---|---|
| *S. aureus* | 7 | 6 | 3 | 3 |
| *276* | | | | |
| *S. aureus* | 8 | 4 | 3 | 3 |
| *070* | | | | |
| *S. epidermidis* | 19 | 8 | 4 | 4 |
| *009* | | | | |
| *S. xylosus* | 11 | 9 | 5 | 5 |
| *008* | | | | |
| *S. saprophyticus* | 19 | 9 | 19 | 12 |
| *030* | | | | |
| *S. haemolyticus* | 19 | 7 | 19 | 12 |
| *131* | | | | |
| *S. intermedius* | 5 | 7 | 2 | 2 |
| *075* | | | | |

L'analyse des résultats montre qu'en détectant l'activité α-glucosidase à l'aide d'un substrat fluorescent à base d'Umbelliferone, il est possible de distinguer les espèces à coagulase positive (*Staphylococcus aureus* et *Staphylococcus intermedius*), des espèces à coagulase négative (*Staphylococcus epidermidis, Staphylococcus xylosus, Staphylococcus saprophyticus* et *Staphylococcus haemolyticus*) en fonction du temps de lecture. En effet, l'hydrolyse du substrat 4-MU-α-GLU par α-glucosidase permet d'obtenir un niveau maximal de fluorescence avant 8 heures d'incubation pour les souches de staphylocoques à coagulase positive et après 10 heures d'incubation pour les souches à coagulase négative.

Pour les trois autres substrats étudiés, il semble difficile de distinguer les espèces de staphylocoques à coagulase positive des staphylocoques à coagulase négative telles que *Staphylococcus epidermidis,* car le délai pour atteindre le niveau de fluorescence maximal pour cette espèce est très proche voir quasi identique de celui fournis par les espèces à coagulase positive.

Cet exemple illustre clairement un des avantages de la détection d'une activité α-glucosidase à l'aide un substrat fluorescent.

### Expérience 6 : Mélange d'inhibiteurs favorisant la détection de l'α-glucosidase avec un substrats enzymatique selon l'invention :

De nombreux travaux ont été réalisés pour améliorer encore la spécificité de nos substrats. Parmi les solutions envisagées, la possibilité d'ajouter des inhibiteurs a abouti à une solution tout à fait étonnante et particulièrement intéressante.

Il a été envisagé certaines combinaisons d'inhibiteurs puisque aucun inhibiteur ne pouvait à lui seul permettre l'amélioration de la détection des Staphylocoques. Parmi celles-ci, une majorité pouvait dissuader l'homme du métier de continuer à essayer de trouver une solution dans cette direction, ce qui est par exemple le cas de :
- La première combinaison est celle de trois inhibiteurs Chlorure de Lithium LiCl (2 g/1), Amphotéricine (0,005 g/l) et Aztréonam (0,008 g/1). La croissance des Staphylocoques est très légèrement affectée par ces trois composés, en revanche il n'y a aucun effet sur les activités enzymatiques détectées (α-glucosidase et β-glucuronidase).
- La deuxième composition du mélange d'inhibiteurs est identique à l'essai précédent, seules les concentrations en LiCl (variant de 3 g/l à 7 g/l) et Aztréonam (variant dans le même temps de 0,008 g/l à 0,003 g/l) sont modifiées simultanément (réalisation d'une gamme de concentrations croisées). Plus la concentration en LiCl augmente et plus la croissance des Staphylocoques diminue. Cet effet n'est pas observé pour l'Aztréonam, l'augmentation de concentration ne perturbe pas la croissance des bactéries. Concernant les activités enzymatiques, il n'y a aucun effet des inhibiteurs (quelque soit la concentration) sur la détection de l'α-glucosidase et de la β-glucuronidase. La sélectivité est améliorée avec l'augmentation de la concentration des inhibiteurs, cependant la fertilité des Staphylocoques est diminuée.
- Le mélange d'inhibiteurs précédent est complété d'une part par la colistine et d'autre part par une augmentation de la concentration en Aztréonam. Cette augmentation, de même que l'addition de colistine dans le milieu, n'a quasiment aucun impact sur la croissance des staphylocoques. La sélectivité est par ailleurs améliorée puisque les espèces critiques identifiées dans les essais précédents sont inhibées dans ces nouvelles conditions. La sensibilité de détection de l'α-glucosidase est très légèrement réduite en présence d'Aztréonam, et ce quelque soit la concentration utilisée (de 0 à 32 mg/l).
- Les performances du milieu, fertilité des Staphylocoques et des non Staphylocoques et expression des activités enzymatiques, sont évaluées en présence du mélange d'inhibiteurs défini dans l'essai précédent (LiCl, Aztréonam, Amphotéricine et Colistine) mais sur un plus large échantillon de souches. L'addition des quatre agents sélectifs dans le milieu n'entraîne qu'une très légère diminution de la croissance des Staphylocoques et reste globalement sans effet sur le niveau d'expression de l'α-glucosidase des *Staphylococcus aureus.* En revanche, la détection/expression de l'activité β-glucuronidase est significativement altérée en présence de ces inhibiteurs. La sélectivité reste encore imparfaite notamment vis-à-vis de *Listeria, Enterococcus faecium, Klebsiella pneumoniae, Acinetobacter baumanii* et *Bacillus.*
- On a ajouté ensuite le composé vibriostatique O/129, augmenté la concentration en Aztréonam (32 mg/l), retiré la colistine du mélange d'inhibiteurs et caractérisé l'activité β-glucosidase. L'expérience ci-après a été réalisée dans le milieu suivant :
   - 20, 1 g/l de peptone,
   - 0,65 g/l de tampon Tris,
   - 14 g/l d' agar,
   - 0,125 g/l de 6-Chloro-3-indoxyl-β-D-glucoside,
   - 0,100 g/l de 5-Bromo-4-chloro-3-indoxyl-N-méthyl-α-D-glucoside,
   - 0,0018 g/l de Chlorure de manganèse, et
   - 4 g/l de pyruvate de sodium.
Par ailleurs, le mélange d'inhibiteurs a la composition suivante :
- LiCI à 5 g/l,
- O/129 à 0,010 g/l,
- Aztréonam à 0,032 g/l, et
- Amphotéricine à 0,005 g/l.

L'ensemble des performances du milieu est étonnamment bonne. En effet, la croissance de toutes les espèces de Staphylocoques est correcte. Par ailleurs, la détection des activités α-glucosidase et β-glucosidase n'est pas affectée par les inhibiteurs et le niveau de sélectivité est satisfaisant puisque la croissance des non Staphylocoques (*Listeria, Acinetobacter, Klebsiella*...) est inhibée.

### Expérience 7 : Amélioration de la sensibilité pour les faibles inocula par ajout de maltose :

L'expérience ci-après a été réalisée dans les milieux contenant :
- 20,1 g/l de peptone,
- 0,65 g/l de tampon Tris,
- 14 g/l d'agar,
- 0,125 g/l de 6-Chloro-3-indoxyl-β-D-glucoside,
- 0,100 g/l de 5-Bromo-4-chloro-3-indoxyl-N-méthyl-α-D-glucoside,
- 0,004 g/l de Chlorure de manganèse,
- 4 g/l de pyruvate de sodium,
- 4 g/l de chlorure de lithium,
- 0,032 g/l d'Aztréonam,
- 0,002 g/l d'Amphotéricine, et
- maltose à une concentration finale de 0 ou 0,1 ou 0,3 ou 0,4 g/l.

Le maltose est solubilisé dans l'eau à une concentration par exemple de 100 g/l, puis un volume suffisant est ajouté aux trois milieux en surfusion pour obtenir les concentrations finales ci-dessus. Les souches de Staphylocoques issues de la collection interne de bioMérieux ont été ensemencées sur chacun des milieux chromogènes par isolement (stries perpendiculaires serrées sur toute la surface de la boîte) à partir d'une suspension à 0,5 MacFarland diluée au 1/30000. Les boîtes ont été incubées à 37°C pendant 48 heures. Les colonies formées ont été examinées visuellement après 18, 24 et 48 heures d'incubation et l'intensité de coloration des colonies a été notée.

Parmi les concentrations testées, les meilleurs résultats sont obtenus avec 100 et 300 mg/l de maltose, ce sont concentrations qui sont donc reportées dans le tableau 10 ci-dessous. Même si les résultats avec 400 mg/l de maltose sont assez bon, il peut y avoir des faux positifs puisque certains autres Staphylocoques coagulase négative possèdent aussi une activité α-glucosidase.

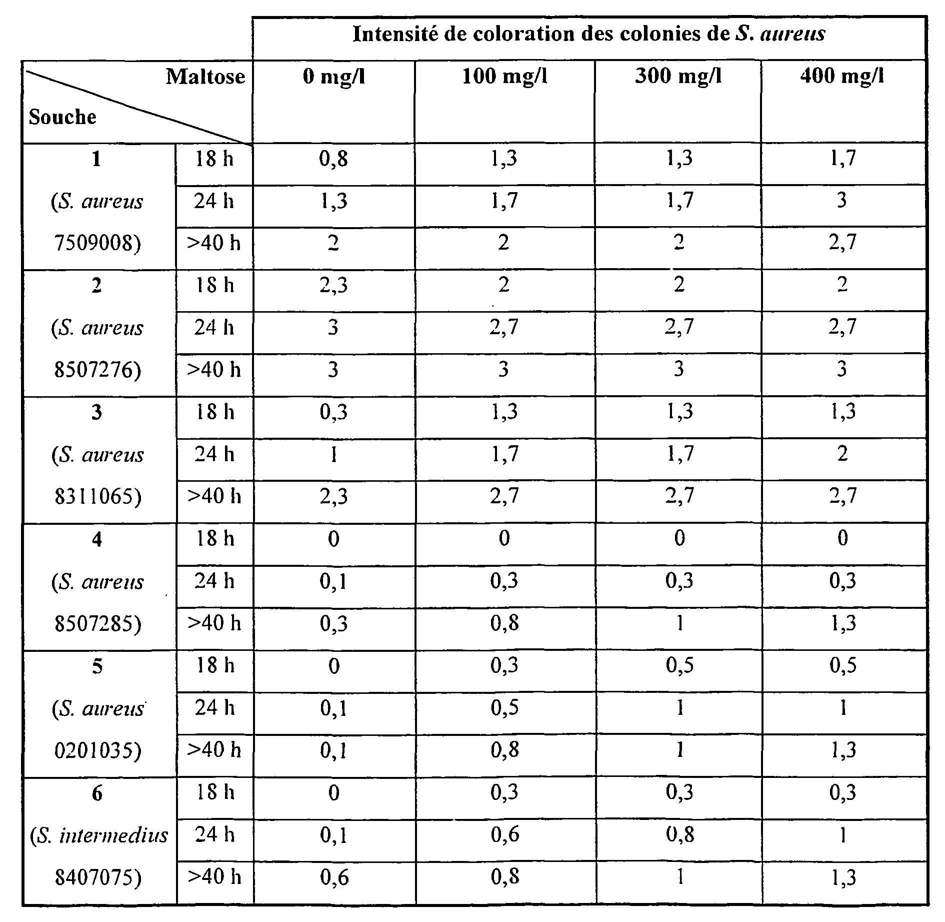

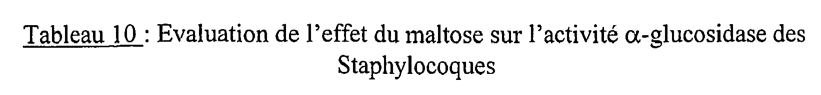

La coloration ainsi que l'intensité de ces colorations ont été notées. L'échelle de lecture est la même que celle utilisée plus haut pour les tableaux 2 et 4.

Le maltose active donc l'expression de l'α-glucosidase des *Staphylococcus aureus* et notamment des souches critiques (3, 4, 5 et 6) permettant ainsi à ces souches de générer des colonies vertes/vertes claires sur les milieux contenant du maltose, alors que les colonies seraient restées blanches en absence de maltose, au risque de rendre une identification non *Staphylococcus aureus* comme expliqué plus haut.

### Divers :

Les substrats chromogènes suivants, révélateur de l'α-glucosidase, peuvent être obtenus auprès des sociétés suivantes :
- 5-Bromo-4-chloro-3-indoxyl-α-D-glucoside auprès de BIOSYNTH située à Staad en Suisse sous la référence B-7230,
- 6-Chloro-3-indoxyl-α-D-glucoside auprès de BIOSYNTH précédemment évoquée et porte la référence C-5015, et
- 6-Bromo-3-indoxyl-α-D-glucoside, dont la synthèse est décrite dans le brevet n° WO 99/50438 de la société Inalco, et
- 2-Naphtyl-α-D-glucopyranoside bien connu de l'homme du métier et pouvant être obtenu auprès de la société Sigma par exemple.

Le substrat fluorescent 4-Methylumbelliféryl-α-D-glucoside, révélateur de l'α-glucosidase, bien connu de l'homme du métier et pouvant être obtenu auprès de la société Sigma par exemple.

En ce qui concerne le 5-Bromo-4-chloro-3-indoxyl-N-méthyl-α-D-glucoside, il peut être synthétisé de la manière suivante :
Le 5-Bromo-4-chloro-1-méthyl-indol-3-yl-α-D-glucoside, formule ci-dessous : 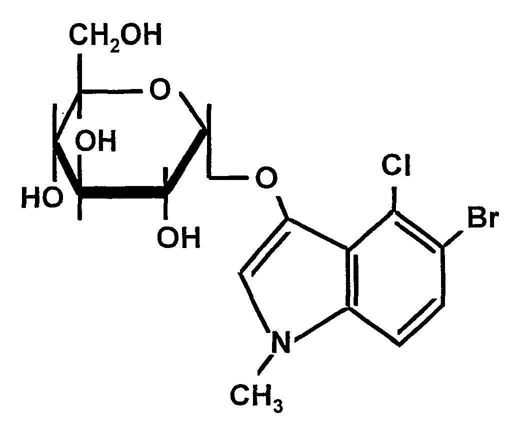 est obtenu à partir du 5-Bromo-4-chloro-3-indolyl acétate ou du 5-Bromo-4-chloro-indolyl-1,3-diacétate.

### Etape 1 : Synthèse du 5-Bromo-4-chloro-N-méthyl-3-indolyl-acétate

3mmol de l'un ou l'autre ester ci-dessus sont dissoutes dans 35 ml de Diéthyl ester anhydre et méthylés sur l'Azote 1 par addition d'un excès de complexe Diazométhane-Trifluorure de Bore dans l'éther. La phase organique est extraite avec de l'acide Chlorydrique 1M, lavée à l'eau et séchée (Na₂SO₄). Le solvant est éliminé et le résidu séché sous vide en présence de Pentoxide de Phosphore pour obtenir 0,62g de produit.

### Etape 2 : Synthèse du 5-Bromo-4-chloro-1-méthyl-3-indolyl-α-D-glucoside

A. L'ester d'acétate N-méthylé obtenu ci-dessus (0,6g, 2mmol) est dissous dans de l'Acétate d'Ethyl anhydre et hydrolysé par addition d'un excès d'Ammoniaque méthanolique sous atmosphère d'Argon. Après 16 heures à température ambiante, le solvant et l'excès d'ammoniaque sont éliminé par évaporation sous pression réduite, le résidu solide est directement glycosilé sans purification supplémentaire.
B. Le 5-Bromo-4-chloro-1-méthyl-indol-3-yl est repris dans du Dichlorométhane et traité à 0°C avec une solution de Glucose penta-acétate dans du Dichlorométhane (1,45g, 5mmol/20ml), puis par du Chlorure d'Etain (2mmol) dissous dans du Dichlorométhane, sous agitation. La réaction est poursuivie pendant une nuit et le produit isolé par agitation avec de l'acide Chlorydrique glacial 1M. Après élimination des sels d'Etain, la phase organique est filtrée sur papier PHASE-SEP et séchée sur MgSO₄. En CCM, deux spots sont détectés sous UV. Après une première séparation sur une colonne de gel de Silice avec du Dichlorométhane-Acétate d'Etyl comme éluant, les deux composés sont séparément déacétylés en présence d'une solution méthanolique de Méthoxide de Sodium pour donner des mélanges d'α et β-glucoside.
C. La forme α apparaît prédominante, le β-glucoside résiduel peut être éliminé par dissolution dans l'eau chaude, refroidie à 37°C et incubée en présence de β-glucosidase. La solution est filtrée et les pigments d'indigo sont extraits à l'Ether. La solution aqueuse résiduelle est évaporée sous pression réduite puis lyophilisée pour obtenir l'α-glucoside (120 mg).

Le substrat chromogène 6-chloro-3-indoxyl-β-D-glucuronide, révélateur de la β-glucuronidase, peut être obtenu auprès de BIOSYNTH toujours située à Staad en Suisse sous la référence C-5050.

Le substrat chromogène 5-Bromo-4-chloro-3-indoxyl-β-D-glucuronide, également révélateur de la β-glucuronidase, peut être obtenu auprès de BIOSYNTH sous la référence B-7400.

Le substrat chromogène 6-Chloro-3-indoxyl-β-D-galactoside, révélateur de la β-galactosidase, peut être obtenu auprès de BIOSYNTH sous la référence C-5000.

Le substrat chromogène 5-Bromo-4-chloro-3-indoxyl-β-D-glucoside, révélateur de la β-glucosidase, peut être obtenu auprès de BIOSYNTH sous la référence B-7250.

## Revendications

1. Milieu de détection de *Staphylococcus aureus* et/ou de Staphylocoques à coagulase positive, qui comporte un milieu de culture de *Staphylococcus aureus* et au moins un substrat enzymatique permettant de mettre en évidence une activité α-glucosidase, **caractérisé par le fait que** le substrat enzymatique permettant de mettre en évidence une activité α-glucosidase est un agent chromogène ou fluorescent et en ce qu'il utilise au moins un inhibiteur qui privilégie la croissance des bactéries du genre *Staphylococcus,* tel que Chlorure de lithium (LiCl), Azide de sodium (NaN₃), Colistine, Amphotéricine, Aztréonam, Colimicine, Chlorure de sodium (NaCl) et Déféroxamine.

2. Milieu, selon la revendication 1, de détection de *Staphylococcus aureus* et de Staphylocoques à coagulase positive, **caractérisé par le fait que** l'agent chromogène ou fluorescent permettant de mettre en évidence une activité α-glucosidase est à base d'Indoxyle ou d'Umbelliférone.

3. Milieu, selon la revendications 1 ou 2, **caractérisé par le fait qu'**il utilise comme agent(s) chromogène(s) :
• 6-Bromo-3-indoxyl-α-D-glucoside,
• 5-Bromo-4-chloro-3-indoxyl-α-D-glucoside,
• 5-Bromo-4-chloro-3-indoxyl-N-méthyl-α-D-glucoside,
• 6-Chloro-3-indoxyl-α-D-glucoside, et/ou
• 4-Méthylumbelliféryl-α-D-glucoside.

4. Milieu, selon la revendication 1, de détection de *Staphylococcus aureus,* **caractérisé par le fait que** l'agent chromogène permettant de mettre en évidence une activité α-glucosidase est à base de Naphtole.

5. Milieu, selon l'une quelconque des revendications 1 ou 4, **caractérisé par le fait qu'**il utilise comme agent chromogène : 2-Naphtyl-α-D-glucopyranoside.

6. Milieu, selon l'une quelconque des revendications 1 à 5, **caractérisé par le fait qu'**il utilise au moins deux substrats enzymatiques différents, préférentiellement deux agents chromogènes, l'un permettant de mettre en évidence une activité αglucosidase et l'autre permettant de détecter une activité osidase (β-glucosidase et/ou β-glucuronidase et/ou β-galactosidase) et/ou estérase (estérase/lipase et/ou phosphatase et/ou sulfatase) et/ou peptidase et/ou coagulase

7. Milieu, selon la revendication 6, **caractérisé par le fait qu'**il utilise deux agents chromogènes, choisis parmi les couples suivants :
• 5-Bromo-4-chloro-3-indoxyl-N-méthyl-α-D-glucoside associé au 5-Bromo-6-chloro-3-indoxyl-β-D-galactoside, ou
• 5-Bromo-4-chloro-3-indoxyl-N-méthyl-α-D-glucoside associé au 6-Chloro-3-indoxyl-β-D-glucuronide, ou
• 6-Chloro-3-indoxyl-α-D-glucoside associé au 5-Bromo-4-chloro-3-indoxyl-β-D-glucuronide, ou
• 6-Chloro-3-indoxyl-α-D-glucoside associé au 5-Bromo-4-chloro-3-indoxyl-β-D-glucoside,

8. Milieu, selon l'une quelconque des revendications 1 à 7, **caractérisé par le fait qu'**il utilise un mélange d'inhibiteurs, comprenant quatre inhibiteurs, qui privilégie la croissance des bactéries du genre *Staphylococcus,* qui sont :
• LiCl,
• O/129,
• Aztréonam, et
• Amphotéricine.

9. Milieu, selon l'une quelconque des revendications 1 à 8, **caractérisé par le fait qu'**il utilise du maltose, préférentiellement à une concentration comprise entre 100 et 300 mg/l.

10. Méthode d'identification de bactérie(s) de l'espèce *Staphylococcus aureus* dans un échantillon, contenant au moins une espèce de bactérie, qui comprend les étapes suivantes :
• inoculer un milieu, selon l'une quelconque des revendications 1 à 9, avec tout ou partie de l'échantillon, et
• incuber le milieu inoculé pour produire des colonies bactériennes de taille suffisante pour observer la coloration de chaque agent chromogène ou la fluorescence de chaque agent fluorescent, ayant réagi, utilisé dans ledit milieu.

11. Méthode, selon la revendication 10, permettant en outre le dénombrement de bactérie(s) de l'espèce *Staphylococcus aureus* dans l'échantillon **caractérisée en ce que** l'on effectue une troisième étape qui consiste à énumérer les colonies identifiées par une coloration ou une fluorescence en relation avec les bactéries du genre *Staphylococcus aureus.*

## Claims

1. A medium for detecting *Staphylococcus aureus* and/or coagulase-positive Staphylococci, which comprises a medium for culturing *Staphylococcus aureus* and at least one enzymatic substrate enabling the revealing of an α-glucosidase activity, **characterised in that** the enzymatic substrate enabling the revealing of an α-glucosidase activity is a chromogenic or fluorescent agent and **in that** it uses at least one inhibitor which promotes the growth of bacteria of the *Staphylococcus* genus, such as Lithium chloride (LiCI), Sodium azide (NaN₃), Colistine, Amphotericin, Aztreonam, Colimicin, Sodium chloride (NaCl) and Deferoxamine.

2. The medium according to Claim 1, for detecting *Staphylococcus aureus* and coagulase-positive Staphylococci, **characterised in that** the chromogenic or fluorescent agent enabling the revealing of an α-glucosidase activity is Indoxyl-based or Umbelliferone-based.

3. The medium according to Claim 1 or 2, **characterised in that** it uses, as a chromogenic agent or agents:
• 6-Bromo-3-indoxyl-α-D-glucoside,
• 5-Bromo-4-chloro-3-indoxyl-α-D-glucoside,
• 5-Bromo-4-chloro-3-indoxyl-N-methyl-α-D-glucoside,
• 6-Chloro-3-indoxyl-α-D-glucoside, and/or
• 4-Methylumbelliferyl-α-D-glucoside.

4. The medium according to Claim 1, for detecting *Staphylococcus aureus,* **characterised in that** the chromogenic agent enabling the revealing of an α-glucosidase activity is Naphthol-based.

5. The medium according to any one of Claims 1 or 4, **characterised in that** it uses as a chromogenic agent: 2-Naphthyl-α-D-glucopyranoside.

6. The medium according to any one of Claims 1 to 5, **characterised in that** it uses at least two different enzymatic substrates, preferably two chromogenic agents, one enabling the revealing of an α-glucosidase activity and the other enabling the detection of an osidase (β-glucosidase and/or β-glucuronidase and/or β-galactosidase) and/or esterase (esterase/lipase and/or phosphatase and/or sulphatase) and/or peptidase and/or coagulase activity.

7. The medium according to Claim 6, **characterised in that** it uses two chromogenic agents, selected from the following pairs:
• 5-Bromo-4-chloro-3-indoxyl-N-methyl-α-D-glucoside associated with 5-Bromo-6-chloro-3-indoxyl-β-D-galactoside, or
• 5-Bromo-4-chloro-3-indoxyl-N-methyl-α-D-glucoside associated with 6-Chloro-3-indoxyl-β-D-glucuronide, or
• 6-Chloro-3-indoxyl-α-D-glucoside associated with 5-Bromo-4-chloro-3-indoxyl-β-D-glucuronide, or
• 6-Chloro-3-indoxyl-α-D-glucoside associated with 5-Bromo-4-chloro-3-indoxyl-β-D-glucoside.

8. The medium according to any one of Claims 1 to 7, **characterised in that** it uses a mixture of inhibitors, including four inhibitors, which promotes the growth of bacteria of the *Staphylococcus* genus, which are:
• LiCl,
• O/129,
• Aztreonam, and
• Amphotericin.

9. The medium according to any one of Claims 1 to 8, **characterised in that** it uses maltose, preferably at a concentration of between 100 and 300mg/l.

10. A method for identifiying a bacterium or bacteria of the *Staphylococcus aureus* species in a sample, containing at least one bacteria species, which includes the following steps:
• inoculating a medium, according to any one of Claims 1 to 9, with all or part of the sample, and
• incubating the inoculated medium in order to produce bacterial colonies of a size which is sufficient to observe the colouring of each chromogenic agent or the fluorescence of each fluorescent agent, having reacted, used in said medium.

11. The method according to Claim 10, furthermore enabling the counting of a bacterium or bacteria of the *Staphylococcus aureus* species in the sample, **characterised in that** a third step is carried out which consists in enumerating the colonies identified by a colouring or a fluorescence in relation to the bacteria of the *Staphylococcus aureus* genus.

## Patentansprüche

1. Milieu zur Feststellung des *Staphylococcus aureus* und/oder positiver Koagulase-Staphylokokken, das ein Kulturmilieu des *Staphylococcus aureus* umfasst und mindestens ein enzymatisches Substrat, das die Feststellung einer α-Glukosidase-Aktivität gestattet, **gekennzeichnet durch** die Tatsache, dass das enzymatische Substrat, das die Feststellung einer α-Glukosidase-Aktivität ermöglicht, ein chromogener oder fluoreszierender Wirkstoff ist und dass dieser mindestens einen Hemmstoff verwendet, der das Wachstum der Bakterien der Gattung des *Staphylococcus* begünstigt, wie zum Beispiel Lithiumchlorid (LiCl), Natriumsäure (NaN₃), Colistin, Amphoterizin, Aztreonam, Colimizin, Natriumchlorid (NaCl) und Deferoxamin.

2. Milieu nach Anspruch 1 zur Feststellung des *Staphylococcus aureus* und positiver Koagulase-Staphylokokken, **gekennzeichnet durch** die Tatsache, dass der chromogene oder fluoreszendierende Wirkstoff, der die Feststellung einer α-Glukosidase-Aktivität gestattet, auf Indoxyl oder Umbelliferon basiert.

3. Milieu nach Anspruch 1 oder 2, **gekennzeichnet durch** die Tatsache, dass es als (einen) chromogene(n) Wirkstoff(e) verwendet:
• 6-Bromo-3-indoxyl-α-D-glukosid,
• 5-Bromo-4-chloro-3-indoxyl-α-D-glukosid,
• 5-Bromo-4-chloro-3-indoxyl-N-methyl-α-D-glukosid,
• 6-Chloro-3-indoxyl-α-D-glukosid, und/oder
• 4-Methylumbelliferyl-α-D-glukosid.

4. Milieu nach Anspruch 1 zur Feststellung des *Staphylococcus aureus,* **gekennzeichnet durch** die Tatsache, dass der chromogene Wirkstoff, der die Feststellung einer α-Glukosidase Aktivität gestattet, auf Naphtol basiert.

5. Milieu nach einem der Ansprüche 1 oder 4, **gekennzeichnet durch** die Tatsache, dass es als chromogenen Wirkstoff 2-Naphtyl-α-D-Glukopyranosid verwendet.

6. Milieu nach einem der Ansprüche 1 bis 5, **gekennzeichnet durch** die Tatsache, dass es mindestens zwei verschiedene enzymatische Substrate verwendet, vorzugsweise zwei chromogene Wirkstoffe, wobei der eine die Feststellung einer α-Glukosidase-Aktivität gewährleistet und der andere die Feststellung einer Osidase- (β-Glukosidase und/oder β-Glukuronidase und/oder β-Galaktosidase) und/oder Esterase- (Esterase/Lipase und/oder Phosphotase und/oder Sulfatase) und/oder Peptidase- und/oder Koagulase-Aktivität.

7. Milieu nach Anspruch 6, **gekennzeichnet durch** die Tatsache, dass es zwei chromogene Wirkstoffe verwendet, ausgewählt aus den folgenden Paaren:
• 5-Bromo-4-chloro-3-indoxyl-N-methyl-α-D-glukosid in Verbindung mit 5-Bromo-6-chloro-3-indoxyl-β-D-galaktosid, oder
• 5-Bromo-4-chloro-3-indoxyl-N-methyl-α-D-glukosid in Verbindung mit 6-Chloro-3-indoxyl-β-D-glukuronid, oder
• 6-Chloro-3-indoxyl-α-D-glukosid in Verbindung mit 5-Bromo-4-chloro-3-indoxyl-β-D-glukuronid, oder
• 6-Chloro-3-indoxyl-α-D-glukosid in Verbindung mit 5-Bromo-4-chloro-3-indoxyl-β-D-glukosid.

8. Milieu nach einem der Ansprüche 1 bis 7, **gekennzeichnet durch** die Tatsache, dass es eine Mischung von Hemmstoffen verwendet, enthaltend vier Hemmstoffe, die das Wachstum von Bakterien der Gattung des *Staphylococcus* begünstigen, wobei diese:
• LiCl,
• O/129,
• Azetreonam, und
• Amphoterizin sind.

9. Milieu nach einem der Ansprüche 1 bis 8, **gekennzeichnet durch** die Tatsache, dass es Maltose verwendet, vorzugsweise bei einer Konzentration zwischen 100 und 300 mg/l.

10. Verfahren zur Bestimmung der Bakterie(n) der Gattung des *Staphylococcus aureus* in einer Stichprobe, enthaltend mindestens eine Gattung der Bakterie, welches die folgenden Schritte umfasst:
• Impfen eines Milieus nach irgendeinem der Ansprüche 1 bis 9 mit der ganzen oder einem Teil der Stichprobe, und
• Inkubieren des geimpften Milieus zur Herstellung von Bakterienkolonien in genügend großer Größe, um die Einfärbung jedes chromogenen Wirkstoffes oder die Fluoreszenz jedes fluoreszierenden Wirkstoffes, die reagiert haben und im besagten Milieu verwendet wurden, zu beobachten.

11. Milieu nach Anspruch 10, welches ferner die Zählung der Bakterie(n) der Gattung des *Staphylococcus aureus* in einer Stichprobe gestattet, **gekennzeichnet dadurch, dass** man einen dritten Schritt durchführt, der darin besteht, die identifizierten Kolonien durch eine Färbung oder eine Fluoreszenz im Verhältnis zu den Bakterien der Gattung des *Staphylococcus aureus* auszuzählen.
